(19) Europäisches Patentamt
European Patent Office
Office européen des brevets

(11) Publication number: **0 446 301 B1**

(12) **EUROPEAN PATENT SPECIFICATION**

(45) Date of publication of patent specification: **23.11.94**

(51) Int. Cl.5: **C07B 37/04**, C07C 15/14, C07C 25/18, C07C 43/164, C07C 43/205, C07D 213/22, C07C 1/26, C07C 17/26, C07C 41/30, C07D 213/127, B01J 31/26, //C07C49/784, C07C69/76,C07C255/51

(21) Application number: **90901266.8**

(22) Date of filing: **13.11.89**

(86) International application number:
**PCT/US89/05218**

(87) International publication number:
**WO 90/06295 (14.06.90 90/14)**

Consolidated with 89420436.1/0376856 (European application No./publication No.) by decision dated 12.10.93.

(54) **PREPARATION OF BIARYL COMPOUNDS.**

(30) Priority: **30.11.88 US 277826**
**03.08.89 US 389022**

(43) Date of publication of application:
**18.09.91 Bulletin 91/38**

(45) Publication of the grant of the patent:
**23.11.94 Bulletin 94/47**

(84) Designated Contracting States:
**AT BE CH DE ES FR GB GR IT LI LU NL SE**

(56) References cited:
**EP-A- 0 012 201**

**The Journal of Organic Chemistry, vol.. 51, no. 14, 11 July 1986 American Chemical Society (US) I Colon et al. Pages 2627-2637**

**Bulletin of the Chemical Society of Japan, vol. 57, no. 7, July 1984 K Takagi et al. Pages 1887-1890**

(73) Proprietor: **EASTMAN CHEMICAL COMPANY**
**100 North Eastman Road**
**Kingsport, TN 37660 (US)**

(72) Inventor: **PUCKETTE, Thomas, Allen**
**306 Jamie Court**
**Longview, TX 75601 (US)**

(74) Representative: **Behrens, Dieter, Dr.-Ing. et al**
**Wuesthoff & Wuesthoff**
**Patent- und Rechtsanwälte**
**Schweigerstrasse 2**
**D-81541 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to the preparation of biaryl compounds from aryl halides. In a particular aspect, the present invention relates to the reductive coupling of aryl halides.

It is known that biphenyl compounds can be produced by the reductive coupling of aryl halides. For example, Chao, et al., in the Journal of Organic Chemistry, Volume 48, at pages 4904-4907 (1983), disclose the reaction of aryl halides with an equivalent amount of a highly activated metal such as nickel powder.

An alternative approach is to activate an aryl halide by a chemical transformation, and then allow the activated aryl halides to couple to form biaryl species. For example, Gilman, et al., in the Journal of the American Chemical Society, Volume 61, at pages 957-959 (1939), demonstrated this approach by the reaction of two equivalents of aryl Grignard reagents with one equivalent of nickel(II) salts to give biaryl compounds. This reaction is believed to proceed through the bis(aryl)nickel(0) species which then decomposes to give the desired biaryl product.

Kumada, et al., in Bulletin of the Chemical Society of Japan, Volume 49, at pages 1958-1969 (1976), have demonstrated that aryl halides can be reacted with a variety of aliphatic Grignard reagents to give alka-aryl products. However, attempts to couple aryl Grignard reagents with aryl halides were successful only with aryl bromides. Attempts to use aryl chlorides gave less than a ten percent yield of desired biaryl products.

Aryl chlorides are frequently more readily available than are the corresponding bromides and iodides. The chlorides are also typically less reactive and are less expensive materials as well. It would, therefore, be desirable to find a means to promote the coupling of aryl chlorides to produce high yields of biaryl compounds.

Such coupling of aryl chlorides has been disclosed by Colon, et al., in U.S. 4,263,466. The authors disclose the use of a metallic reducing agent such as zinc, magnesium, or manganese in a dipolar, aprotic solvent such as dimethylformamide with a catalyst containing a nickel compound in combination with triaryl organophosphines and alkali metal halide promoters. The reducing metal converts the nickel salts into highly reactive zerovalent nickel compounds which promote the coupling of the aryl halides and regenerate the nickel salts which can be reduced again to the zerovalent state, thereby maintaining the catalytic cycle.

In later publications by the same authors (see Colon, et al., in Journal of Organic Chemistry, Vol. 51 at pages 2627-2637 (1986), it is indicated that bidentate ligands (as opposed to the monodentate triaryl organophosphines disclosed in '466) are not effective for the nickel/reducing metal-promoted reductive coupling reaction. Takagi, et al., in the Bulletin of the Chemical Society of Japan, at pages 1887-1890 (1984) have made similar observations. See especially Run Number 14 reported at page 1888.

In accordance with the present invention, it has surprisingly been found that aryl chlorides can be reductively coupled employing bidentate ligands to produce biaryl compounds in high yield. By contacting aryl halides with a catalyst comprising a nickel compound, at least one bidentate phosphorus-containing ligand selected from a specified group and a reducing metal, high yields of biaryl compounds are obtained.

The practice of the present invention allows for the ready preparation of biaryl derivatives from aryl chloride starting materials. Aryl chlorides are generally preferred starting materials as they are more accessible on a commercial basis and are generally less expensive than the corresponding aryl bromides or aryl iodides.

In accordance with the present invention, there is provided a method for the preparation of biaryl compounds of the structure:

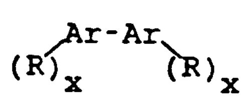

wherein Ar is an aromatic moiety having in the range of 4 up to 20 carbon atoms, each R is independently selected from alkyl, aryl, -F, $-NR'_2$, -CN, -CHO, -OR', -OCO-R', -COO-R',

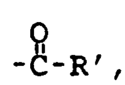

$-SO_2$-R', $-SO_3$R', or -NR'COR':
wherein R' is a hydrocarbyl or heteroaryl radical having up to 20 carbon atoms, and x is an integer falling in

the range of 0 up to 8, depending on the size of the aromatic ring, Ar, and with the proviso that there be no more than one substituent ortho to the Ar-Ar bond.

The invention method comprises contacting an aromatic halide having the structure:

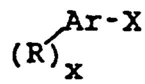

wherein Ar, R and x are as defined above and X is a halogen; under conditions suitable for the formation of the desired biaryl compound in the presence of at least 0.001 equivalents of a nickel catalyst comprising:

I) an anhydrous nickel compound,

II) at least one bidentate phosphorus-containing ligand selected from the group consisting of:

(i) 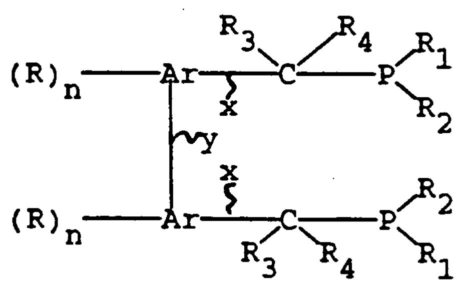

wherein each Ar is independently selected from aromatic ring compounds having 6 up to 14 carbon atoms, e.g., phenyl, naphthyl, phenanthryl or anthracenyl;

the x bonds and the y bonds are attached to adjacent carbon atoms on the ring structures;

each R, when present as a substituent, is independently selected from alkyl, aryl, -F, $-NR'_2$, -CN, -CHO, -OR', -OCO-R', -COO-R',

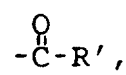

$-SO_2-R'$, $-SO_3-R'$, or -NR'COR':

wherein R' is a hydrocarbyl or heteroaryl radical having up to 20 carbon atoms;

n is a whole number in the range of 0-4 where Ar is phenyl; 0-6 where Ar is naphthyl; and 0-8 where Ar is phenanthryl or anthracenyl;

each $R_1$ and $R_2$ is independently selected from alkyl, aryl, aralkyl, alkaryl or cycloaliphatic radicals, unsubstituted or substituted with amino, amido, sulfonic acids, oxycarbonyl, hydroxyl and alkoxy groups;

each $R_3$ and $R_4$ is independently selected from hydrogen and the $R_1$ substituents;

each of the above alkyl groups or moieties is straight or branched chain of 1-20 carbons;

each aryl group contains 4-20 ring carbons; and

each cycloaliphatic group contains from 4-8 ring carbons;

(ii) 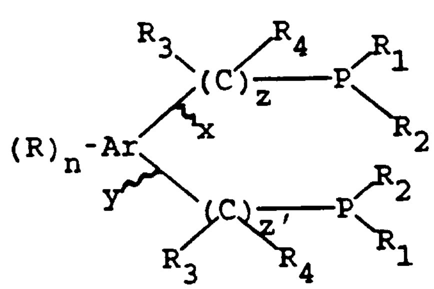

wherein Ar, x, y, R, n, $R_1$, $R_2$, $R_3$, and $R_4$ are each as defined above, and each of z and z' can independently vary between 0 and 4, with the proviso that z + z' is at least 2;

(iii) 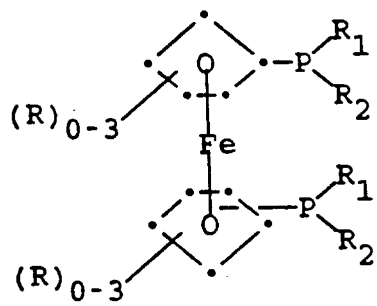

wherein R, $R_1$, and $R_2$ are as defined above; and

(iv) 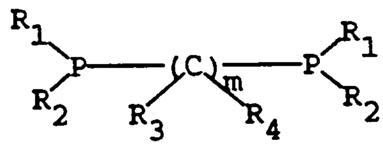

wherein $R_1$, $R_2$, $R_3$, and $R_4$ are as defined above; and m is a whole number which can vary from 4 up to 8; preferably from 5 up to 8;

as well as mixtures of any two or more of said type (i), (ii), (iii), or (iv) organophosphines; and

III) at least one reducing metal selected from the group consisting of zinc, magnesium and maganese,

wherein said contacting is carried out in a polar, aprotic, solvent system for a time and under conditions suitable for the formation of the desired biaryl compound.

Reaction temperatures employed in the practice of the present invention can vary widely. Typically, reaction temperatures fall in the range of 0 up to 250 °C, with reaction temperatures in the range of 25 up to 120 °C being preferred.

Reaction times contemplated for the practice of the present invention can vary widely. One of skill in the art can readily determine that amount of time which is sufficient to allow formation of the desired biaryl or heterobiaryl compounds. Typically, reaction times will fall in the range of 0.1 up to 24 hours or longer, with reaction times in the range of 0.5 up to 16 hours being preferred.

Aryl halides contemplated for use in the practice of the present invention are compounds have the general structure:

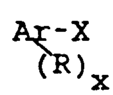

wherein Ar is an aromatic moiety having in the range of 4 up to 20 carbon atoms, each R is independently selected from alkyl, aryl, -F, $-NR'_2$, -CN, -CHO, -OR', -OCO-R',

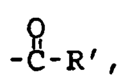

$-SO_2R'$, $SO_3R'$, or NR'COR': wherein R' is a hydrocarbyl or heteroaryl radical having up to 20 carbon atoms; X is a halogen and x is an integer falling in the range of 0 up to 8, depending on the size of the aromatic ring, Ar, and with the further proviso that there be no more than one substituent ortho to the halogen moiety. Exemplary aryl halides contemplated for use in the practice of the present invention include:

2-chlorotoluene,
2-bromotoluene,
4-chlorotoluene,
4-bromotoluene,
2-chloro-4-methylnaphthalene,
2-bromo-4-methylnaphthalene,
4-chloroanisole,
4-bromoanisole,
4-chlorophenylacetate,
4-bromophenylacetate,
2-chlorobenzyl(2-methoxy)ethyl ether,
2-bromobenzyl(2-methoxy)ethyl ether,
2-chlorobenzyl methyl ether,
2-bromobenzyl methyl ether,
2-chlorobenzyl ethyl ether,
2-bromobenzyl ethyl ether,
2-chlorothiophene,
2-bromothiophene,
2-chloropyridine,
2-bromopyridine,
2-chloro-3-methylnaphthalene,
2-bromo-3-methylnaphthalene,
1-chloro-2-methylnaphthalene,
1-bromo-2-methylnaphthalene,
1-methyl-2-chloronaphthalene,
1-methyl-2-bromonaphthalene,
4-fluoro-2-chlorotoluene,
4-fluoro-2-bromotoluene,
4-(N-ethyl-N-acetyl)amino-2-chlorotoluene,
4-(N-ethyl-N-acetyl)amino-2-bromotoluene,
6-(N-ethyl-N-acetyl)amino-2-chlorotoluene,
6-(N-ethyl-N-acetyl)amino-2-bromotoluene,

and the like, as well as mixtures of any two or more thereof.

Presently preferred monohalides contemplated for use in the practice of the present invention include compounds having the structural formula:

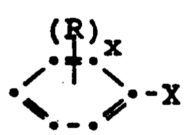

(I)

wherein one or more of the carbon atoms of the benzene ring in formula (I) is optionally replaced by N;
wherein X is a halogen selected from the group consisting of Cl, Br and I;
R is a monovalent radical selected from alkyl, aryl, -F, $-NR'_2$, -CN, -CHO, -OR', -OCO-R, -COO-R',

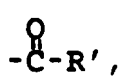

$-SO_2-R'$, $-SO_3-R'$, or -NR'COR':

wherein R' is a hydrocarbyl or heteroaryl radical having up to 20 carbon atoms,and x is an integer having values of 0 to 4 with the proviso that no more than one R is in a position ortho to the X-containing ring carbon atom.

Additional compounds contemplated by the above structural formula are those wherein one or more of the carbon atoms of the benzene ring is replaced by N. Especially preferred compounds contemplated by

the above formula are those wherein X is Cl.

Examples of preferred monohalides which satisfy structural formula (I) include:

2-chlorotoluene,
2-chloropyridine,
4-fluoro-2-chlorotoluene,
2-chlorobenzyl methyl ether,
2-chlorobenzyl ethyl ether,
4-(N-ethyl-N-acetyl)amino-2-chlorotoluene,
6-(N-ethyl-N-acetyl)amino-2-chlorotoluene,
4-chlorotoluene,
4-chloroanisole,
2-chlorobenzyl(2-methoxy)ethyl ether,
as well as mixtures of any two or more thereof.

A wide range of nickel compounds are suitable for use in the practice of the present invention, so long as the nickel compounds employed are essentially water-free. The nickel(II) halide salts are a convenient source of nickel as such compounds are readily available in anhydrous form. Alternatively, hydrates of such compounds can be employed if an appropriate means of water removal, e.g., azeotropic distillation, is employed prior to contacting the nickel species with the reducing metal and aryl halide: Those of skill in the art recognize that a wide variety of nickel compounds can be used in addition to the nickel(II) halides, e.g., nickel nitrates, sulfates, phosphates, oxides, carbonates, carboxylates, acetylacetonate and the like, as well as Ni(O) complexes such as, for example, bis(1,5-cyclooctadienyl)nickel(O), nickel(O) tetracarbonyl, and the like.

The nickel(II) halides are presently preferred because of their ready availability in anhydrous form (or, alternatively, the case with which the hydrates forms such compounds can be dehydrated), and because the presence of halides in the reaction mixture appears to promote the coupling reaction. Especially preferred are nickel chloride and nickel bromide.

Suitable ratios of nickel to aryl halides can vary widely. Molar ratios in the range of 0.0001 up to 0.5:1 are generally suitable. Ratios in the range of 0.01 up to 0.2:1 are preferred, with molar ratios in the range of 0.03 up to 0.1 being most preferred because good conversions are obtained at reasonable reaction rates.

Optionally added to the catalyst composition are inorganic salt promoters. When used, preferred promoters include alkali, alkaline earth, zinc, magnesium, manganese and aluminum halides, or mixtures thereof. Bromides are particularly preferred. The amount of promoter, when used, can range from 0.1 to 1000 moles per gram atom of nickel with 1 to 100 moles of promoter being preferred.

The presently most preferred promoters include alkali metal iodides, alkali metal bromides and alkali metal chlorides.

Organophosphines contemplated for use in the practice of the present invention are compounds having the structures set forth above as (i), (ii), (iii), or (iv).

Exemplary compounds contemplated by structure (i) include compounds having the structural formula:

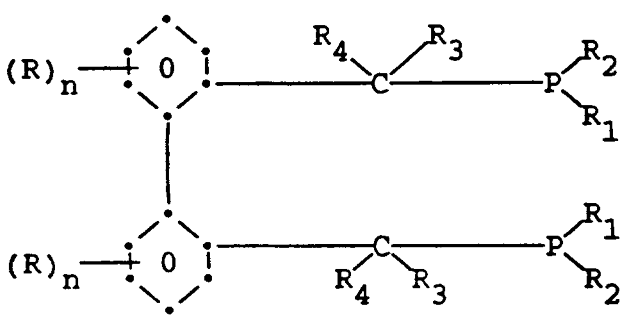

wherein R, $R_1$, $R_2$, $R_3$, $R_4$, and n are as defined above; including such compounds as

2,2'-bis(diphenylphosphinomethyl)-1,1'-biphenyl;
2,2'-bis(dibenzylphosphinomethyl)-1,1'-biphenyl;
2,2'-bis(phenylbenzylphosphinomethyl)-1,1'-biphenyl; and
2,2'-bis(diisobutylphosphinomethyl)-1,1'-biphenyl.

Exemplary compounds contemplated by structure (i) also include compounds having the structural formula:

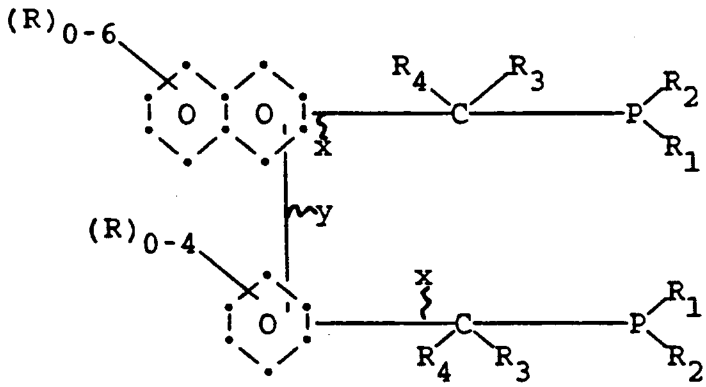

wherein R, $R_1$, $R_2$, $R_3$, $R_4$, x and y are defined above; such as, for example 2-(diphenylphosphinomethyl)-1-[2-(diphenylphosphino-methyl)phenyl]naphthalene.

Exemplary compounds contemplated by structure (i) also include compounds having the structural formula:

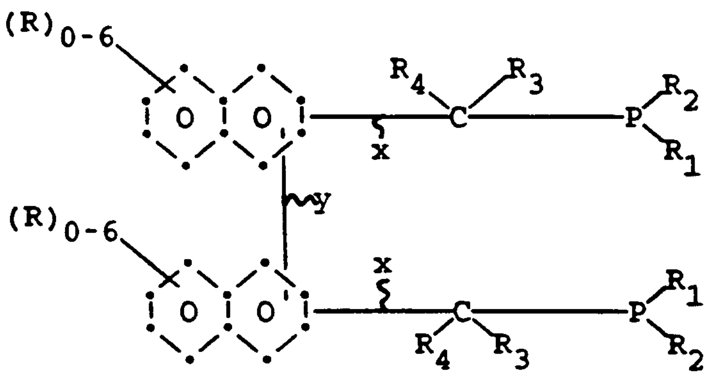

wherein R, $R_1$, $R_2$, $R_3$, $R_4$, x and y are as defined above; such as, for example 2,2'-bis(diphenylphosphinomethyl)-1,1'-binaphthyl.

Exemplary compounds contemplated by structure (ii) include compounds having the structural formula:

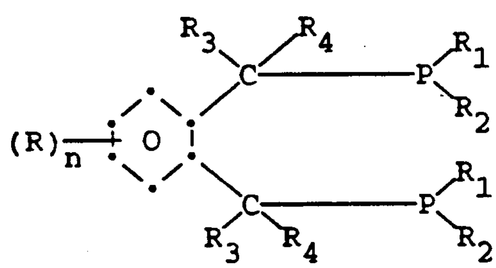

wherein R, $R_1$, $R_2$, $R_3$, $R_4$ and n are as defined above; such as, for example $\alpha,\alpha'$-bis(diphenylphosphino)ortho-xylene.

Exemplary compounds contemplated by structure (ii) also include compounds having the structural formula:

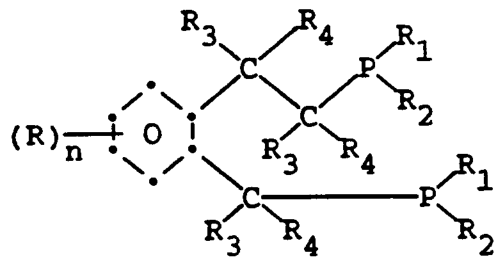

wherein R, $R_1$, $R_2$, $R_3$, $R_4$ and n are as defined above; such as, for example α,β'-bis(diphenylphosphino)-2-ethyltoluene.

Exemplary compounds contemplated by structure (ii) also include compounds having

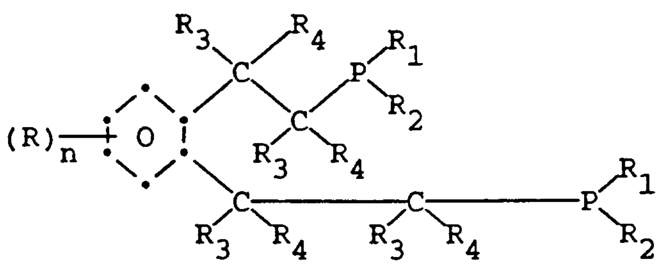

wherein R, $R_1$, $R_2$, $R_3$, $R_4$ and n are as defined above; such as, for example 1,2-bis[2-(diphenylphosphino)ethyl]benzene.

Exemplary compounds contemplated by structure (iii) include: 1,1'-bis(diphenylphosphino)ferrocene, and the like.

Exemplary compounds contemplated by structure (iv) include:
1,4-bis(diphenylphosphino)butane,
1,5-bis(diphenylphosphino)pentane, and
1,6-bis(diphenylphosphino)hexane;
as well as mixtures of any two or more thereof. Also contemplated are mixtures of any two or more of said type (i), (ii), (iii) or (iv) organophosphines.

The molar ratio of organophosphine to nickel compound employed in the practice of the present invention can vary widely. Typically, such molar ratio will fall within the range of 0.5:1 up to 20:1, with ratios in the range of 1:1 up to 10:1 preferred. Ratios in the range of 1:1 up to 3:1 are presently most preferred because little added benefit is seen in the use of large excesses of organophosphine(s).

Optional coordinating ligands employed in combination with the above-described bidentate organophosphines are bidentate ligands containing at least one nitrogen atom as part of an aromatic ring structure. Such bidentate ligands include bipyridine, a $C_1$ up to $C_6$ dialkylamino pyridine, phenanthroline or 2-picolinic acid, and the like.

When the optional use of mixtures of bidentate organophosphine and bidentate ligand containing at least one nitrogen atom as part of an aromatic ring structure are employed as the coordinating ligand, molar ratios of the bidentate organophosphine to the bidentate ligand containing at least one nitrogen atom as part of an aromatic ring structure can vary widely, for example, in the range of 0.1:1 up to 20:1, with ratios in the range of 0.5 up to 10 preferred. Ratios in the range of 1:1 up to 2:1 are presently most preferred because little added benefit is observed when large excesses of the bidentate ligand containing at least one nitrogen atom as part of an aromatic ring structure are employed.

Solvents suitable for use in the practice of the present invention include dipolar, aprotic solvents such as solvents, such as N,N-dimethylacetamide, N,N-dimethylformamide, 1-methyl-2-pyrrolidinone, tetramethylurea, dimethylsulfoxide, sulfolane, and the like. If desired these dipolar, aprotic solvents can be mixed with lower polarity inert solvents, such as saturated aliphatic hydrocarbons, including pentanes, hexanes, dodecanes and the like; aromatic hydrocarbons, such as benzene, toluene, xylenes and the like; and saturated aliphatic and cycloaliphatic ethers, such as, diethyl ether, diglyme, tetrahydrofuran and the like.

It is preferred that all solvents use in the practice of this invention be anhydrous.

Although magnesium and manganese metals can be used, zinc metal is the presently preferred reducing metal for use in the invention process for coupling aryl monochlorides.

The molar ratio of reducing metal to aryl halide employed in the practice of the present invention can vary widely. Typically, such molar ratio will fall within the range of about 0.01:1 up to 20:1, with ratios in the range of about 0.2:1 up to 10:1 preferred. Ratios in the range of about 0.4:1 up to 5:1 are presently most preferred because it is desired to minimize the quantity of reducing metal which must be removed from the reaction mixture and little added benefit is obtained when large excesses of reducing metal are employed.

The molar ratio of reducing metal to nickel employed in the practice of the present invention can vary widely. Typically, such molar ratios will fall within the range of 1 up to 1000:1, with ratios in the range of 10 up to 500:1 being preferred. Ratios in the range of 40 up to 100:1 are presently preferred for the same reasons as stated in the preceding paragraph.

Preparation of the novel catalyst composition is carried out conveniently by mixing the aforementioned nickel compound, ligand(s), promoter, and reducing metal(s) in the dipolar, aprotic solvent under an inert atmosphere and heating to a temperature in the range of 25° to 80°C.

The present invention will now be described in greater detail by reference to the following non-limiting examples.

## EXAMPLES

All reactions were conducted under an inert atmosphere. Glassware was dried prior to use and flushed with nitrogen. Commercial materials were used when possible and the following were used as purchased: Anhydrous nickel(II) bromide, sodium bromide, anhydrous dimethylformamide, and zinc powder (-325 mesh). Organophosphine ligands were purchased or prepared by methods known by those of skill in the art.

### Typical Experimental Procedure

Nickel(II) bromide (0.22 g, 1 mmol), the desired phosphine ligand (1.5 to 3 mmol), sodium bromide (3 g), anhydrous dimethylformamide (DMF; 15 mL), zinc powder (3 g, 45 mmol) and an aryl halide (20 mmoles) were combined under a nitrogen atmosphere and heated to 70°C for 14 to 16 hours. The mixture was cooled to ambient and analyzed by conventional chromatography techniques. In those cases where the DMF solvent may obscure one of the products, the reaction mixture was partitioned between aqueous ammonium chloride and ethyl acetate. The organic phase was then analyzed by normal procedures.

Results of numerous runs with different aryl halides and different phosphine ligands are summarized in Tables I, II and III.

Table I

| Reductive Dimerization of 2-Chlorotoluene Using Linear Bidentate Phosphines, $(Ph)_2P-(CH2)_m-P(Ph)_2$* | | | | |
|---|---|---|---|---|
| Phosphine m = | Mmole Ligand | [P]/[Ni][1] | % Convn of 2-CT[2] | % Selectivity, 2,2'-Bitolyl |
| 1 | 1.5 | 3 | 0 | 0 |
| 2 | 1.5 | 3 | 24.9 | 97.8 |
| 3 | 1.5 | 3 | 9.4 | 100 |
| 4 | 1.5 | 3 | 100 | 94.9 |
| 5 | 1.5 | 3 | 100 | 96.1 |
| 6 | 1.5 | 3 | 100 | 95.4 |

*All runs were carried out at 70°C for 16 hours using 1 mmol $NiBr_2$, 30 mmol zinc, 3.0 grams of NaBr, 20 mmol of 2-CT in 15 mL DMF and ligand as noted.
1) Atomic ratio of phosphorus to nickel.
2) 2-CT = 2-chlorotoluene

These results demonstrate that for linear bidentate phosphines of the type (iv), m must be at least 4, i.e., there must be at least 4 carbon atoms in the chain between the phosphine units. Chain lengths longer than 4 carbon atoms are seen to be particularly effective.

Table II

| Reductive Dimerization of Halotoluenes to Bitolyls* | | | | |
|---|---|---|---|---|
| Aryl Halide | % Convn | % Selectivity to | | % Expected Dimer |
| | | Toluene | Dimers | |
| 2-Chlorotoluene | 100 | <0.5 | 100 | 98.9[1] |
| 2-Bromotoluene | 100 | 4.85 | 95.15 | 97.7[1] |
| 3-Iodotoluene | 100 | 10.8 | 89.2 | 80.4[2] |

*All runs were conducted at 70 °C for 16 hours using 1 mmol $NiBr_2$, 30 mmol zinc, 20 mmol aryl halide, 3.0 g of NaBr in DMF (15 mL). The ligand for all of these runs was 2,2'-bis(diphenylphosphinomethyl) -1,1'-biphenyl (BISBI; 1.5 mmol).
[1]Expected dimeric product is 2,2'-bitolyl.
[2]Expected dimeric product is 3,3'-bitolyl.

These results demonstrate that aryl chlorides are preferred starting materials for the invention reductive coupling reaction because they give the desired products in high yields with very high selectivity, without producing significant levels of reduced mono-aromatic product (i.e., toluene) or isomerized product (e.g., 2,3'-bitolyl).

Table III

The Reductive Dimerization of Substituted Aryl Halides to Substituted Biaryl Derivatives*

| Aryl Halide | Ligand[1] | % Convn | % Selectivity[2] H-Aryl | % Selectivity[2] Dimers | % Expected Isomeric Dimer[3] |
|---|---|---|---|---|---|
| 4-Bromo-anisole | OXYL | 100 | 11.4 | 88.6 | 62.7 |
| 4-Bromo-anisole | BISBI | 99.7 | 4.6 | 95.4 | 75.3 |
| 4-Chloro-benzotri-fluoride | OXYL | 85.9 | 3.7 | 96.3 | 98.7 |
| 4-Chloro-benzotri-fluoride | BISBI | 100 | 2 | 98 | 98.7 |
| 4-Chloro-benzoni-trile | OXYL | 100 | 16.5 | 83.5 | 79.7 |
| 4-Chloro-benzoni-trile | BISBI | 100 | 5.2 | 94.8 | 72.9 |
| Methyl-3-chloro-benzoate | OXYL | 100 | 7.9 | 92.1 | 93.3 |
| Methyl-3-chloro-benzoate | BISBI | 100 | 3.8 | 96.2 | 89.2 |
| 2-Chloro-aceto-phenone | OXYL | 88.9 | 43.2 | 56.8 | 66.6 |
| 2-Chloro-aceto-phenone | BISBI | 100 | 9.5 | 90.5 | 54.5 |

Table III (Continued)

The Reductive Dimerization of Substituted Aryl Halides to Substituted Biaryl Derivatives*

| Aryl Halide | Ligand[1] | % Convn | % Selectivity[2] H-Aryl | % Selectivity[2] Dimers | % Expected Isomeric Dimer[3] |
|---|---|---|---|---|---|
| 4-Chloro-benzalde-hyde | OXYL | 100 | 60 | 40 | 100 |
| 4-Chloro-benzalde-hyde | BISBI | 100 | 19.7 | 80.3 | 100 |
| 1-Bromo-2-methyl-naphtha-lene | BISBI | 28.8 | 3.6 | 96.4 | 100 |
| 1-Bromo-3-fluoro-benzene | BISBI | 100 | <0.5 | 99.5 | 100 |

*All runs were carried out at 70°C for 16 hours using 1 mmol $NiBr_2$, 30 mmol zinc, 20 mmol aryl halide, 3.0 g NaBr, 1.5 mmol of ligand in DMF (15 mL).

1) OXYL = α,α'-bis(diphenylphosphino)ortho-xylene.

BISBI = 2,2'-bis(diphenylphosphinomethyl)-1,1'-biphenyl

2) H-Aryl is the reduction product of the aryl halide wherein the halogen has been replaced with a hydrogen.

Dimers refers to all aromatic-aromatic coupling products.

## Table III (Continued)

**The Reductive Dimerization of Substituted Aryl Halides to Substituted Biaryl Derivatives***

*3) Expected isomeric dimer of:

4-bromoanisole is 4,4'-dimethoxybiphenyl;

4-chlorobenzotrifluoride is 4,4'-bis(trifluoromethyl)biphenyl;

4-chlorobenzonitrile is 4,4'-dicyanobiphenyl;

methyl-3-chlorobenzoate is the dimethyl ester of 3,3'-diphenyldicarboxylic acid;

2-chloroacetophenone is 2,2'-diacetylbiphenyl;

4-chlorobenzaldehyde is 4,4'-diphenyldicarboxaldehyde;

1-bromo-2-methylnaphthalene is 2,2'-dimethyl-1,1'-binaphthyl; and

1-bromo-3-fluorobenzene is 3,3'-difluorobiphenyl.

The data in Table III demonstrate that a wide variety of functional groups are compatible with the invention process.

## Claims

1. A method for coupling aryl and heteroaryl monohalides having 4 to 20 carbon atoms to produce biaryl or hetero-biaryl compounds, of the structure:

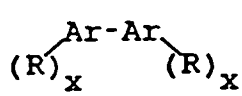

wherein Ar is an aromatic moiety having in the range of 4 up to 20 carbon atoms, each R is independently selected from alkyl, aryl, -F, $-NR'_2$, -CN, -CHO, -OR', -OCO-R', -COO-R',

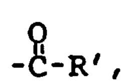

$-SO_2-R'$, $-SO_3R'$, or -NR'COR':

wherein R' is a hydrocarbyl or heteroaryl radical having up to 20 carbon atoms, and x is an integer falling in the range of 0 up to 8, depending on the size of the aromatic ring, Ar, and with the proviso that there be no more than one substituent ortho to the Ar-Ar bond; said method comprising contacting said monohalide having the structure :

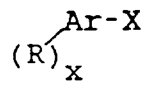

wherein Ar, R and x are as defined above and X is a halogen; with a catalytic combination comprising:

(I) an anhydrous nickel compound, wherein the ratio of gram atoms of nickel to moles of monohalide is in the range of 0.0001 up to 0.5;

(II) at least one bidentate phosphorus-containing ligand selected from the group consisting of

(i)

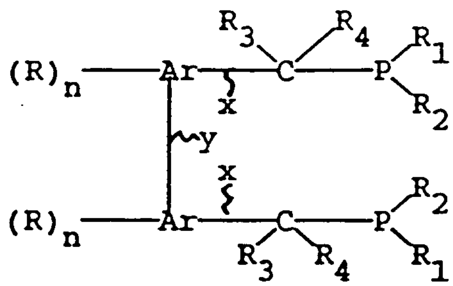

wherein each Ar is independently selected from aromatic ring compounds having 6 up to 14 carbon atoms;

the x bonds and the y bonds are attached to adjacent carbon atoms on the ring structures;

each R, when present as a substituent, is independently selected from alkyl, aryl, -F, $-NR'_2$, -CN, -CHO, -OR', -OCO-R', -COO-R',

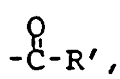

$-SO_2-R'$, $-SO_3R'$, or -NR'COR':

wherein R' is a hydrocarbyl or heteroaryl radical having up to 20 carbon atoms;

n is an integer in the range of 0-4 where Ar is phenyl; 0-6 where Ar is naphthyl; and 0-8 where Ar is phenanthryl or anthracenyl;

each R1 and R2 is independently selected from alkyl, aryl, aralkyl, alkaryl or cycloaliphatic radicals unsubstituted or substituted with amino, amido, sulfonic acids, oxycarbonyl, hydroxyl or alkoxy groups

each $R_3$ and $R_4$ is independently selected from hydrogen and the $R_1$ substituents;

each of the above alkyl groups or moieties is straight or branched chain of 1-20 carbon atoms;

each aryl group contains 4-20 ring carbon atoms; and

each cycloaliphatic group contains from 4-8 ring carbons;

(ii)

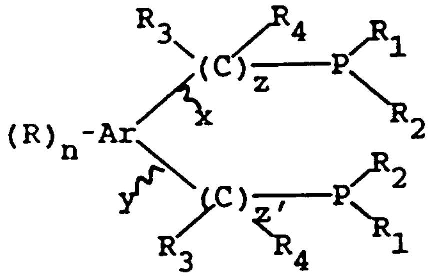

wherein Ar, x, y, R, n, $R_1$, $R_2$, $R_3$ and $R_4$ are each as defined above, and each of z and z' can independently vary between 0 and 4, with the proviso that z + z' is at least 2;

(iii)

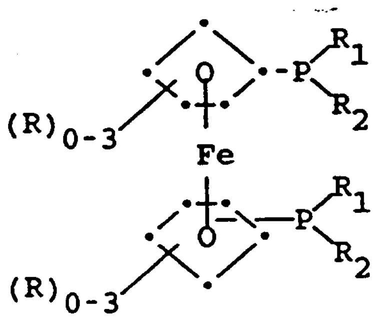

wherein R, $R_1$ and $R_2$ are as defined above; and

(iv)

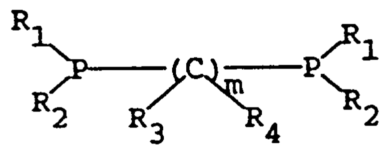

wherein $R_1$, $R_2$, $R_3$ and $R_4$ are as defined above; and m is a whole number which can vary from 4 up to 8;

as well as mixtures of any two or more thereof; wherein the amount of ligand is in the range of 0.5 up to 20 moles per gram atom of nickel; and

(III) at least one reducing metal selected from the group consisting of zinc, magnesium and manganese; wherein the molar ratio of reducing metal to reactant monohalide falls in the range of 0.01 up to 20:1;

wherein said contacting is carried out in the presence of a polar, aprotic solvent at a temperature in the range of 0 up to 250 °C for a time sufficient to form said biaryl or heterobiaryl compounds.

2. The method in accordance with Claim 1 wherein said bidentate ligand has the structural formula:

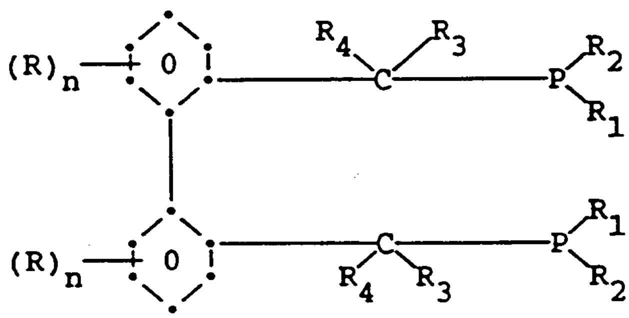

wherein R, $R_1$, $R_2$, $R_3$, $R_4$ and n are as defined above.

3. The method in accordance with Claim 2 wherein said bidentate ligand is selected from the group consisting of:
2,2'-bis(diphenylphosphinomethyl)-1,1'-biphenyl;
2,2'-bis(dibenzylphosphinomethyl)-1,1'-biphenyl;
2,2'-bis(phenylbenzylphosphinomethyl)-1,1'-biphenyl; and
2,2'-bis(diisobutylphosphinomethyl)-1,1'-biphenyl.

4. The method in accordance with Claim 1 wherein said bidentate ligand has the structural formula:

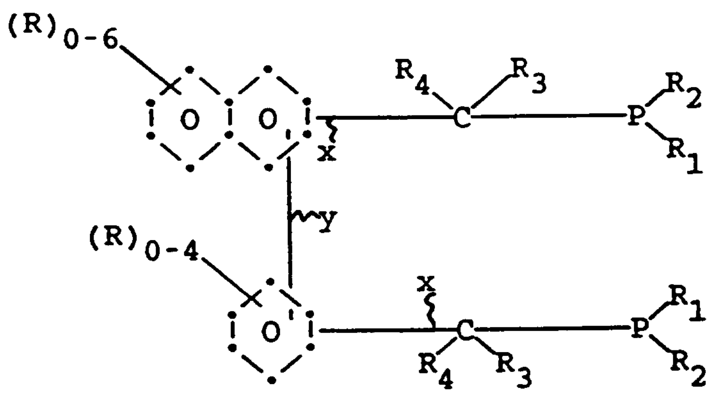

wherein R, $R_1$, $R_2$, $R_3$, $R_4$, x and y are as defined above.

5. The method in accordance with Claim 4 wherein said bidentate ligand is 2-(diphenylphosphinomethyl)-1-[2-(diphenylphosphinomethyl)phenyl]naphthalene.

6. The method in accordance with Claim 1 wherein said bidentate ligand has the structural formula:

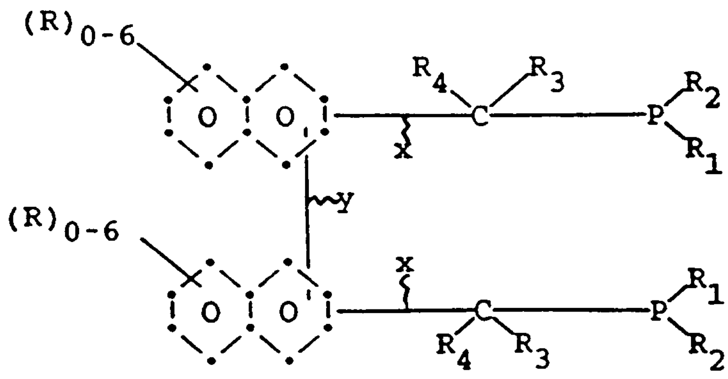

wherein R, $R_1$, $R_2$, $R_3$, $R_4$, x and y are as defined above.

7. The method in accordance with Claim 6 wherein said bidentate ligand is 2,2'-bis-(diphenylphosphinomethyl)-1,1'-binaphthyl.

8. The method in accordance with Claim 1 wherein said bidentate ligand has the structural formula:

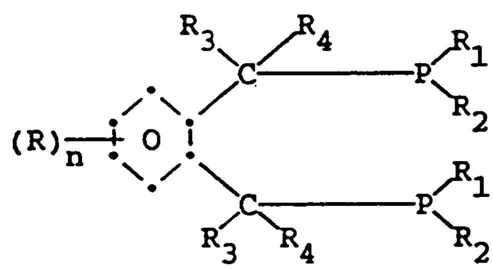

wherein R, $R_1$, $R_2$, $R_3$, $R_4$ and n are as defined above.

9. The method in accordance with Claim 8 wherein said bidentate ligand is $\alpha,\alpha'$-bis(diphenylphosphino)-ortho-xylene.

10. The method in accordance with Claim 1 wherein said bidentate ligand has the structural formula:

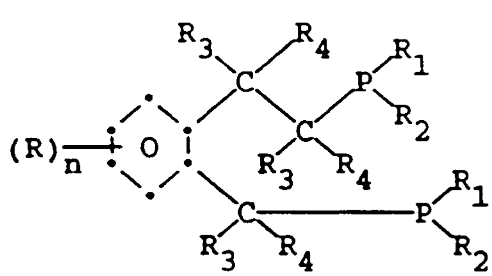

wherein R, $R_1$, $R_2$, $R_3$, $R_4$ and n are as defined above.

11. The method in accordance with Claim 10 wherein said bidentate ligand is $\alpha,\beta'$-bis(diphenylphosphino)-2-ethyltoluene.

**12.** The method in accordance with Claim 1 wherein said bidentate ligand has the structural formula:

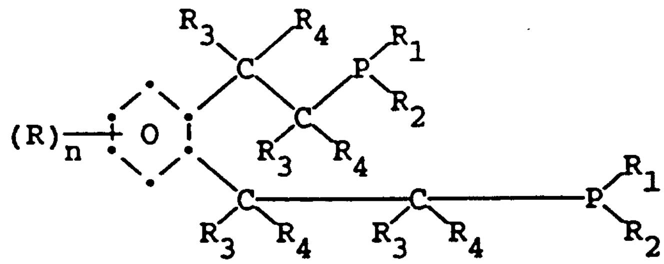

wherein R, $R_1$, $R_2$, $R_3$, $R_4$ and n are as defined above.

**13.** The method in accordance with Claim 12 wherein said bidentate ligand is 1,2-bis[2-(diphenylphosphino)ethyl]benzene.

**14.** The method in accordance with Claim 1 wherein said bidentate ligand is 1,1'-bis(diphenylphosphino)-ferrocene.

**15.** The method in accordance with Claim 1 wherein said bidentate ligand has the structural formula:

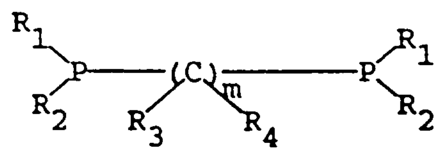

wherein $R_1$, $R_2$, $R_3$ and $R_4$ are as defined above; and wherein m is a whole number which can vary from 4 up to 8.

**16.** The method in accordance with Claim 15 wherein said bidentate ligand is selected from the group consisting of:
1,4-bis(diphenylphosphino)butane,
1,5-bis(diphenylphosphino)pentane,
1,6-bis(diphenylphosphino)hexane,
as well as mixtures of two or more thereof.

**17.** The method in accordance with any of Claims 1 to 16 wherein said catalytic combination further comprises at least one bidentate ligand containing at least one nitrogen atom as part of an aromatic ring structure.

**18.** The method in accordance with Claim 17 wherein said bidentate ligand containing at least one nitrogen atom is selected from 2,2'-bipyridine, a $C_1$ up to $C_6$ dialkylamino pyridine, phenanthroline or 2-picolinic acid.

**19.** The method in accordance with Claim 1 wherein said reducing metal is zinc.

**20.** The method in accordance with Claim 1 wherein the nickel compound is nickel chloride.

**21.** The method in accordance with Claim 1 wherein at least 0.1 moles per gram atom of nickel of an inorganic salt is added as a promoter.

**22.** The method in accordance with Claim 21 wherein the inorganic salt is selected from the group consisting of: an alkali metal iodide, an alkali metal bromide, an alkali metal chloride, as well as mixtures of any two or more thereof.

**23.** The method in accordance with Claim 1 wherein the aprotic solvent is N,N-dimethylacetamide.

**24.** The method in accordance with Claim 1 wherein the aprotic solvent is N,N-dimethylformamide.

**25.** The method in accordance with Claim 1 wherein the aprotic solvent is 1-methyl-2-pyrrolidinone.

**26.** The method in accordance with Claim 1 wherein the monohalide has the formula:

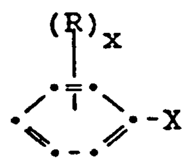

(I)

wherein one or more of the carbon atoms of the benzene ring in formula (I) is optionally replaced by N; wherein X is a halogen selected from the group consisting of Cl, Br and I;
R is a monovalent radical selected from alkyl, aryl, -F, $-NR'_2$, -CN, -CHO, -OR', -OCO-R', -COO-R',

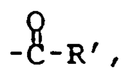

$-SO_2$-R', $-SO_3$-R', or -NR'COR':
wherein R' is a hydrocarbyl or heteroaryl radical having up to 20 carbon atoms, and x is an integer having values of 0 to 4 with the proviso that no more than one R is in a position ortho to the X-containing ring carbon atom.

**27.** The method according to Claim 26 wherein X is Cl.

**28.** The method according to Claim 27 wherein said monohalide is selected from the group consisting of:
2-chlorotoluene,
2-chloropyridine,
4-fluoro-2-chlorotoluene,
2-chlorobenzyl methyl ether,
2-chlorobenzyl ethyl ether,
4-(N-ethyl-N-acetyl)amino-2-chlorotoluene
6-(N-ethyl-N-acetyl)amino-2-chlorotoluene
4-chlorotoluene,
4-chloroanisole,
2-chlorobenzyl(2-methoxy)ethyl ether,
as well as mixtures of any two or more thereof.

**29.** A method as claimed in Claim 26 wherein R is methyl and x is 1.

**30.** A method as claimed in Claim 1 wherein the temperature is from 25 °C to 120 °C.

**31.** The method in accordance with Claim 30 wherein the ratio of gram atoms of nickel to moles of monohalide falls in the range of 0.01 up to 0.2:1; the amount of bidentate phosphorus-containing ligand falls in the range of 1 up to 10 moles per gram atom of nickel; and the molar ratio of reducing metal to reactant monohalide falls in the range of 0.2 up to 10:1.

**32.** The method in accordance with Claim 30 wherein the ratio of gram atoms of nickel to moles of monohalide falls in the range of 0.03 up to 0.1:1; the amount of bidentate phosphorus-containing ligand falls in the range of 1 up to 5 moles per gram atom of nickel; and the molar ratio of reducing metal to

reactant monohalide falls in the range of 0.4 up to 5:1.

**33.** A catalytically active combination comprising:
(I) an anhydrous nickel compound,
(II) at least one bidentate phosphorus-containing ligand selected from the group consisting of

(i)

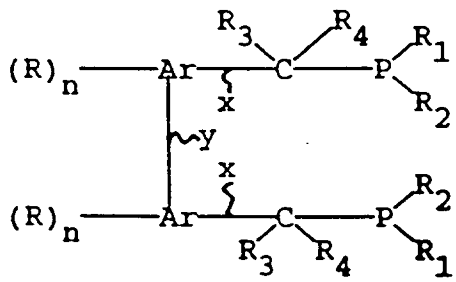

wherein each Ar is independently selected from aromatic ring compounds having 6 up to 14 carbon atoms;

the x bonds and the y bonds are attached to adjacent carbon atoms on the ring structures;

each R, when present as a substituent, is independently selected from alkyl, aryl, -F, $-NR'_2$, -CN, -CHO, -OR', -OCO-R', -COO-R',

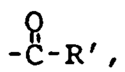

$-SO_2-R'$, $-SO_3-R'$, or -NR'COR':

wherein R' is a hydrocarbyl or heteroaryl radical having up to 20 carbon atoms;

n is an integer in the range of 0-4 where Ar is phenyl; 0-6 where Ar is naphthyl; and 0-8 where Ar is phenanthryl or anthracenyl;

each R1 and R2 is independently selected from alkyl, aryl, aralkyl, alkaryl or cycloaliphatic radicals unsubstituted or substituted with amino, amido, sulfonic acids, oxycarbonyl, hydroxyl or alkoxy groups

each $R_3$ and $R_4$ is independently selected from hydrogen and the $R_1$ substituents;

each of the above alkyl groups or moieties is straight or branched chain of 1-20 carbons;

each aryl group contains 4-20 ring carbons; and

each cycloaliphatic group contains from 4-8 ring carbons;

(ii)

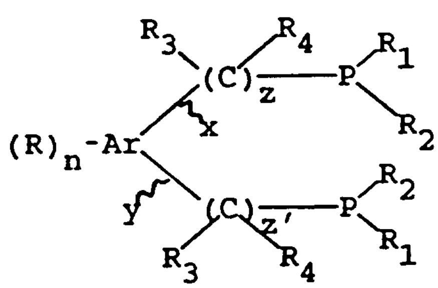

wherein Ar, x, y, R, n, $R_1$, $R_2$, $R_3$, and $R_4$ are each as defined above, and each of z and z' can independently vary between 0 and 4, with the proviso that z + z' is at least 2;

(iii)

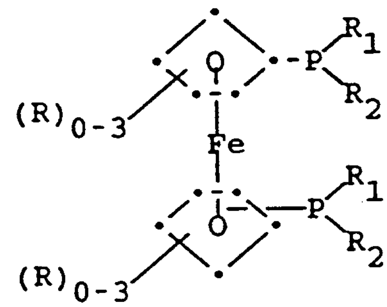

wherein R, $R_1$, and $R_2$ are as defined above; and

(iv)

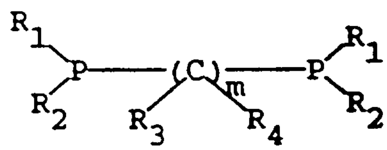

wherein $R_1$, $R_2$, $R_3$ and $R_4$ are as defined above; and wherein m is a whole number which varies from 5 up to 8;

as well as mixtures of any two or more of said type (i), (ii), (iii), or (iv) organophosphines; and

(III) at least one reducing metal selected from the group consisting of zinc, magnesium and manganese;

wherein the amount of bidentate phosphorus-containing ligand falls within the range of 0.5 up to 20 moles per gram atom of nickel; and wherein the amount of reducing metal falls within the range of 1 up to 1000 moles per mole of nickel.

## Patentansprüche

1. Verfahren zur Verknüpfung von Aryl- und Heteroaryl-Monohalogeniden mit 4 bis 20 Kohlenstoffatomen, um Diaryl- oder Hetero-Diarylverbindungen zu erhalten, mit der Struktur:

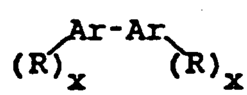

worin Ar ein aromatischer Rest mit etwa 4 bis zu 20 Kohlenstoffatomen ist, wobei jedes R unabhängig voneinander ausgewählt ist aus Alkyl, Aryl, -F, $-NR'_2$, -CN, -CHO, -OR', -OCO-R', -COO-R',

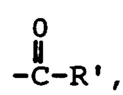

$-SO_2-R'$, $-SO_3R'$ oder -NR'COR':

worin R' ein Kohlenwasserstoff- oder Heteroaryl-Rest mit bis zu 20 Kohlenstoffatomen ist und x eine ganze Zahl ist im Bereich von 0 bis zu 8, abhängig von der Größe des aromatischen Ringes, Ar, und mit der Maßgabe, daß es nicht mehr als einen Substituenten in Ortho-Stellung zu der Ar-Ar-Bindung gibt; dieses Verfahren umfaßt das Inkontaktbringen des Monohalogenids mit der Struktur:

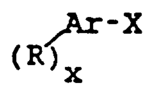

worin Ar, R und x wie zuvor angegeben sind und X ein Halogen ist, mit einer katalytischen Kombination umfassend:

(I) eine wasserfreie Nickelverbindung, wobei das Verhältnis Grammatome Nickel zu Mole Monohalogenid im Bereich von 0,0001 bis zu 0,5 ist;

(II) mindestens einen zweizähnigen Phosphor-enthaltenden Liganden, ausgewählt aus der Gruppe bestehend aus

(i)

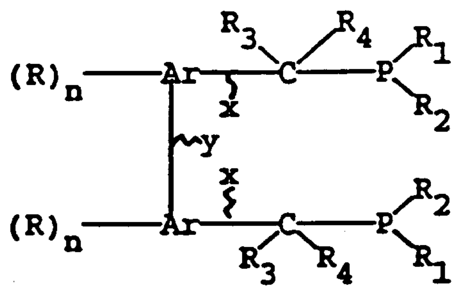

worin jedes Ar jeweils unabhängig voneinander ausgewählt ist aus aromatischen Ringverbindungen mit 6 bis zu 14 Kohlenstoffatomen;

die x-Bindungen und die y-Bindungen sind mit benachbarten Kohlenstoffatomen der Ringstrukturen verknüpft;

jedes R, soweit es als Substituent vorhanden ist, ist unabhängig voneinander ausgewählt aus Alkyl, Aryl, -F, $-NR'_2$, -CN, -CHO, -OR', -OCO-R', -COO-R',

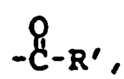

$-SO_2-R'$, $-SO_3R'$, oder -NR'COR':

worin R' ein Kohlenwasserstoff- oder Heteroaryl-Rest mit bis zu 20 Kohlenstoffatomen ist;

n ist eine ganze Zahl im Bereich von 0-4, wenn Ar Phenyl ist; 0-6, wenn Ar Naphthyl ist; und 0-8, wenn Ar Phenanthryl oder Anthracenyl ist;

jedes R1 und R2 ist unabhängig voneinander ausgewählt aus Alkyl, Aryl, Aralkyl, Alkaryl oder cycloaliphatischen Resten nicht-substituiert oder substituiert mit Amino-, Amido-, Sulfonsäure-, Oxycarbonyl-, Hydroxyl- oder Alkoxygruppen

jedes $R_3$ und $R_4$ ist unabhängig voneinander ausgewählt aus Wasserstoff und den $R_1$-Substituenten;

jede der vorstehend genannten Alkylgruppen oder Reste ist geradkettig oder verzweigtkettig mit 1-20 Kohlenstoffatomen;

jede Arylgruppe enthält 4-20 Ringkohlenstoffatome; und

jede cycloaliphatische Gruppe enthält von 4-8 Ringkohlenstoffe;

(ii)

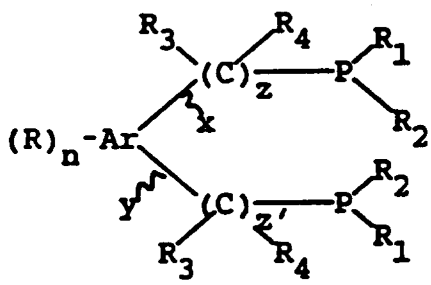

worin Ar, x, y, R, n, $R_1$, $R_2$, $R_3$ und $R_4$ jeweils wie zuvor angegeben sind und z und z' jeweils unabhängig voneinander zwischen 0 und 4 variieren können mit der Maßgabe, daß z + z' mindestens 2 ist;

(iii)

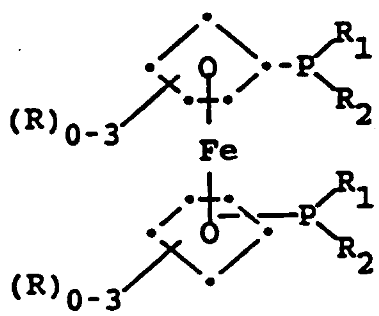

worin R, $R_1$ und $R_2$ wie zuvor angegeben sind; und

(iv)

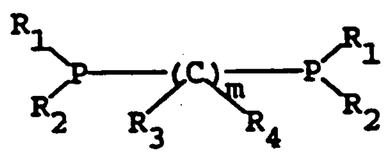

worin $R_1$, $R_2$, $R_3$ und $R_4$ wie zuvor angegeben sind und m eine ganze Zahl ist, die zwischen 4 bis zu 8 variieren kann;

sowie Mischungen von beliebigen zwei oder mehreren davon; wobei die Menge des Liganden im Bereich von 0,5 bis zu 20 Mole pro Grammatom Nickel liegt; und

(III) mindestens ein reduzierendes Metall, ausgewählt aus der Gruppe bestehend aus Zink, Magnesium und Mangan, wobei das Mol-Verhältnis reduzierendes Metall zu Monohalogenid-Reaktant in den Bereich von 0,01 bis zu 20:1 fällt;

wobei das Inkontaktbringen in Gegenwart eines polaren aprotischen Lösungsmittels bei einer Temperatur im Bereich von 0 bis zu 250 °C über eine ausreichende Zeit zur Bildung der Diaryl- oder Heterodiaryl-Verbindungen durchgeführt wird.

2. Verfahren nach Anspruch 1, wobei der zweizähnige Ligand die Strukturformel:

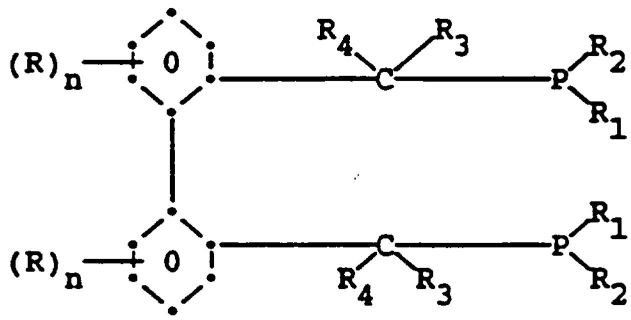

hat, worin R, $R_1$, $R_2$, $R_3$, $R_4$ und n wie zuvor angegeben sind.

3. Verfahren nach Anspruch 2, wobei der zweizähnige Ligand ausgewählt ist aus der Gruppe bestehend aus:
2,2'-Bis(diphenylphosphinomethyl)-1,1'-biphenyl;
2,2'-Bis(dibenzylphosphinomethyl)-1,1'-biphenyl;
2,2'-Bis(phenylbenzylphosphinomethyl)-1,1'-biphenyl und
2,2'-Bis(diisobutylphosphinomethyl)-1,1'-biphenyl.

4. Verfahren nach Anspruch 1, wobei der zweizähnige Ligand die Strukturformel:

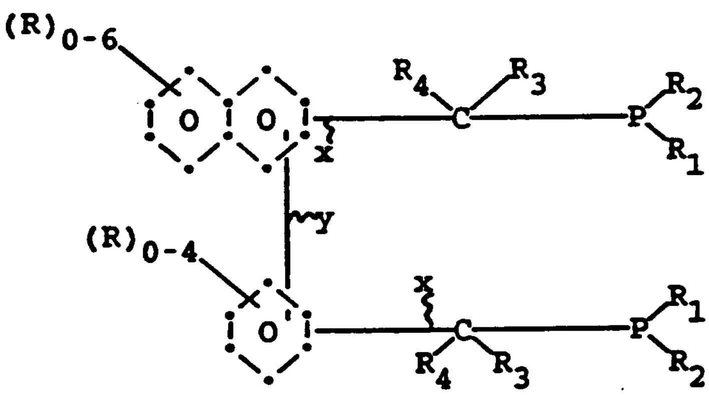

hat, worin R, $R_1$, $R_2$, $R_3$, $R_4$, x und y wie zuvor angegeben sind.

5. Verfahren nach Anspruch 4, wobei der zweizähnige Ligand 2-(Diphenylphosphinomethyl)-1-[2-(diphenylphosphinomethyl)phenyl]-naphthalin ist.

6. Verfahren nach Anspruch 1, wobei der zweizähnige Ligand die Strukturformel:

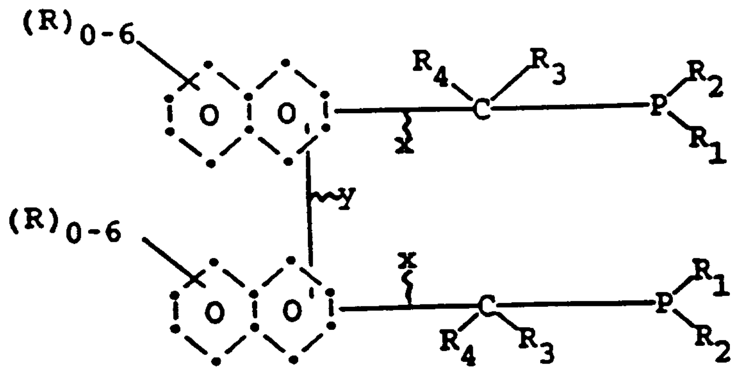

hat, worin R, $R_1$, $R_2$, $R_3$, $R_4$, x und y wie zuvor angegeben sind.

7. Verfahren nach Anspruch 6, wobei der zweizähnige Ligand 2,2'-Bis(diphenylphosphinomethyl)-1,1'-binaphthyl ist.

8. Verfahren nach Anspruch 1, wobei der zweizähnige Ligand die folgende Strukturformel:

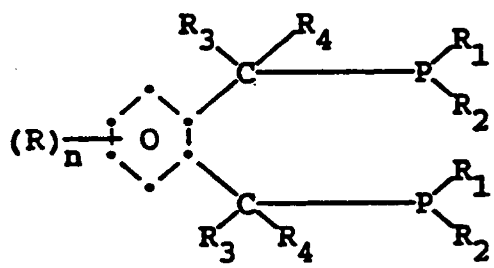

hat, worin R, $R_1$, $R_2$, $R_3$, $R_4$ und n wie zuvor angegeben sind.

9. Verfahren nach Anspruch 8, wobei der zweizähnige Ligand $\alpha,\alpha'$-Bis(diphenylphosphino)ortho-xylol ist.

10. Verfahren nach Anspruch 1, wobei der zweizähnige Ligand die Strukturformel:

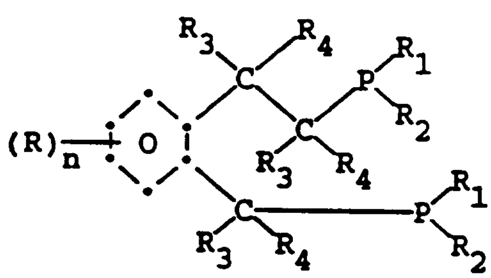

hat, worin R, $R_1$, $R_2$, $R_3$, $R_4$ und n wie zuvor angegeben sind.

11. Verfahren nach Anspruch 2, wobei der zweizähnige Ligand $\alpha,\beta'$-Bis(diphenylphosphino)-2-ethyltoluol ist.

**12.** Verfahren nach Anspruch 1, wobei der zweizähnige Ligand die Strukturformel:

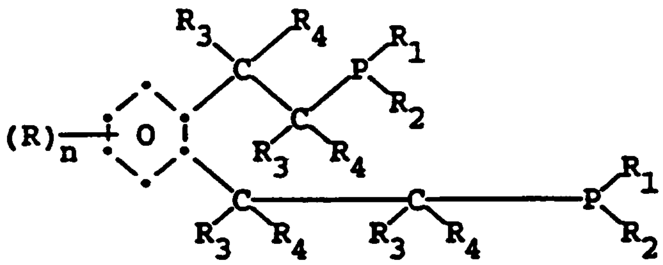

hat, worin R, $R_1$, $R_2$, $R_3$, $R_4$ und n wie zuvor angegeben sind.

**13.** Verfahren nach Anspruch 12, wobei der zweizähnige Ligand 1,2-Bis[2-diphenylphosphino)ethyl]benzol ist.

**14.** Verfahren nach Anspruch 1, wobei der zweizähnige Ligand 1,1'-Bis(diphenylphosphino)ferrocen ist.

**15.** Verfahren nach Anspruch 1, wobei der zweizähnige Ligand die Strukturformel:

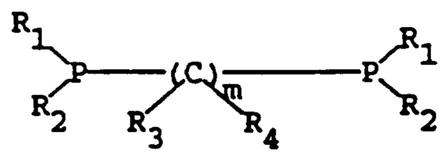

hat, worin $R_1$, $R_2$, $R_3$ und $R_4$ wie zuvor angegeben sind und worin m eine ganze Zahl ist, die von 4 bis zu 8 variieren kann.

**16.** Verfahren nach Anspruch 15, wobei der zweizähnige Ligand ausgewählt ist aus der Gruppe bestehend aus:

1,4-Bis(diphenylphosphino)butan,
1,5-Bis(diphenylphosphino)pentan,
1,6-Bis(diphenylphosphino)hexan,

sowie Mischungen von zwei oder mehreren davon.

**17.** Verfahren nach einem der Ansprüche 1 bis 16, wobei die katalytische Kombination weiterhin mindestens einen zweizähnigen Liganden umfaßt, der mindestens ein Stickstoffatom als Teil einer aromatischen Struktur enthält.

**18.** Verfahren nach Anspruch 17, wobei der zweizähnige Ligand, der mindestens ein Stickstoffatom enthält, ausgewählt ist aus 2,2'-Bipyridin, ein $C_1$- bis zu $C_6$-Dialkylaminopyridin, Phenanthrolin oder 2-Picolinsäure.

**19.** Verfahren nach Anspruch 1, wobei das reduzierende Metall Zink ist.

**20.** Verfahren nach Anspruch 1, wobei die Nickelverbindung Nickelchlorid ist.

**21.** Verfahren nach Anspruch 1, wobei mindestens ein Mol pro Grammatom Nickel eines anorganischen Salzes dem Promoter hinzugegeben wird.

**22.** Verfahren nach Anspruch 21, wobei das anorganische Salz ausgewählt ist aus der Gruppe bestehend aus: ein Alkalimetalliodid, ein Alkalimetallbromid, ein Alkalimetallchlorid, sowie Mischungen von beliebigen zwei oder mehreren davon.

23. Verfahren nach Anspruch 1, wobei das aprotische Lösungsmittel N,N-Dimethylacetamid ist.

24. Verfahren nach Anspruch 1, wobei das aprotische Lösungsmittel N,N-Dimethylformamid ist.

25. Verfahren nach Anspruch 1, wobei das aprotische Lösungsmittel 1-Methyl-2-pyrrolidinon ist.

26. Verfahren nach Anspruch 1, wobei das Monohalogenid die Formel hat:

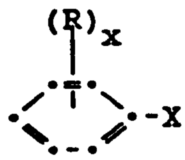

(I)

worin eines oder mehrere der Kohlenstoffatome des Benzolrings in der Formel (I) gegebenenfalls durch N ersetzt ist;
worin X ein Halogenid ist, ausgewählt aus der Gruppe bestehend aus Cl, Br und I;
R ist ein einwertiger Rest, ausgewählt aus Alkyl, Aryl, -F, $-NR'_2$, -CN, -CHO, -OR' -OCO-R', -COO-R',

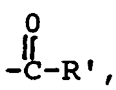

$-SO_2-R'$, $-SO_3-R'$ oder -NR'COR':
worin R' ein Kohlenwasserstoff- oder Heteroaryl-Rest ist mit bis zu 20 Kohlenstoffatomen und x ist eine ganze Zahl mit Werten von 0 bis 4 mit der Maßgabe, daß nicht mehr als ein R in einer Ortho-Position zu dem X-enthaltenden Ringkohlenstoffatom ist.

27. Verfahren nach Anspruch 26, wobei X Cl ist.

28. Verfahren nach Anspruch 27, wobei das Monohalogenid ausgewählt ist aus der Gruppe bestehend aus:
2-Chlortoluol,
2-Chlorpyridin,
4-Fluor-2-chlortoluol,
2-Chlorbenzylmethylether,
2-Chlorbenzylethylether,
4-(N-Ethyl-N-acetyl)amino-2-chlortoluol
6-(N-Ethyl-N-acetyl)amino-2-chlortoluol
4-Chlortoluol,
4-Chloranisol,
2-Chlorbenzyl(2-methoxy)ethylether,
sowie Mischungen von beliebigen zwei oder mehreren davon.

29. Verfahren nach Anspruch 26, wobei R Methyl ist und x gleich 1 ist.

30. Verfahren nach Anspruch 1, wobei die Temperatur von 25°C bis 120°C ist.

31. Verfahren nach Anspruch 30, wobei das Verhältnis Gramm-Atome Nickel zu Mole Monohalogenid in den Bereich von 0,01 bis zu 0,2:1 fällt; die Menge des zweizähnigen Phosphor-enthaltenden Liganden fällt in den Bereich von 1 bis zu 10 Molen pro Grammatom Nickel und das Molverhältnis reduzierendes Metall zu Monohalogenid-Reaktant fällt in den Bereich von 0,2 bis zu 10:1.

**32.** Verfahren nach Anspruch 30, wobei das Verhältnis Grammatom Nickel zu Mole Monohalogenid in den Bereich von 0,03 bis zu 0,1:1 fällt; die Menge an zweizähnigem Phosphor-enthaltendem Liganden fällt in den Bereich von 1 bis zu 5 Molen pro Gramm-Atom Nickel und das Molverhältnis reduzierendes Metall zu Monohalogenid-Reaktant fällt in den Bereich von 0,4 bis zu 5:1.

**33.** Eine katalytisch aktive Kombination, umfassend:

(I) eine wasserfreie Nickelverbindung,

(II) mindestens ein zweizähniger Phosphor-enthaltender Ligand, ausgewählt aus der Gruppe bestehend aus

(i)

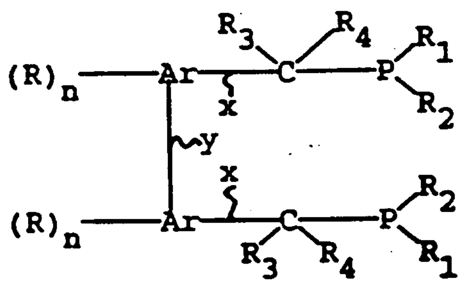

worin jedes Ar jeweils unabhängig voneinander ausgewählt ist aus aromatischen Ringverbindungen mit 6 bis zu 14 Kohlenstoffatomen;

die x-Bindungen und die y-Bindungen sind mit benachbarten Kohlenstoffatomen der Ringstruktur verknüpft;

jedes R, soweit als Substituent vorhanden, ist unabhängig voneinander ausgewählt aus Alkyl, Aryl, -F, $-NR'_2$, -CN, -CHO, -OR', -OCO-R', -COO-R',

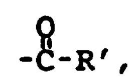

$-SO_2-R'$, $-SO_3-R'$, oder -NR'COR':

worin R' ein Kohlenwasserstoff- oder Heteroaryl-Rest ist mit bis zu 20 Kohlenstoffatomen;

n ist eine ganze Zahl im Bereich von 0-4, wenn Ar Phenyl ist; 0-6, wenn Ar Naphthyl ist; und 0-8, wenn Ar Phenanthryl oder Anthracenyl ist;

jedes R1 und R2 ist jeweils unabhängig voneinander ausgewählt aus Alkyl, Aryl, Aralkyl, Alkaryl oder cycloaliphatischen Resten unsubstituiert oder substituiert mit Amino-, Amido-, Sulfonsäuren, Oxycarbonyl-, Hydroxyl- oder Alkoxygruppen

jedes $R_3$ und $R_4$ ist unabhängig voneinander ausgewählt aus Wasserstoff und dem $R_1$-Substituenten;

jede der vorstehend genannten Alkylgruppen oder Reste ist geradkettig oder verzweigtkettig mit 1-20 Kohlenstoffatomen;

jede Arylgruppe enthält 4-20 Ringkohlenstoffe; und

jede cycloaliphatische Gruppe enthält von 4-8 Ringkohlenstoffe;

(ii)

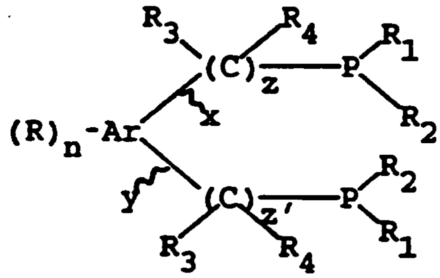

worin Ar, x, y, R, n, $R_1$, $R_2$, $R_3$ und $R_4$ jeweils wie zuvor angegeben sind und jedes z und z' unabhängig voneinander zwischen 0 und 4 variieren kann mit der Maßgabe, daß z + z' mindestens 2 ist;

(iii) 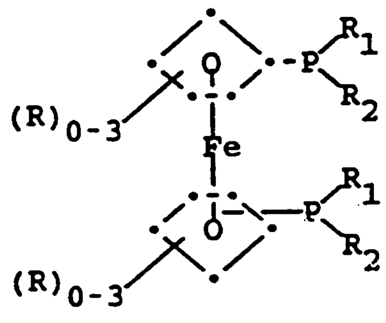

worin R, $R_1$ und $R_2$ wie zuvor angegeben sind; und

(iv) 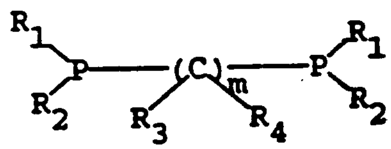

worin $R_1$, $R_2$, $R_3$ und $R_4$ wie zuvor angegeben sind und worin m eine ganze Zahl ist, die von 5 bis zu 8 variiert;

sowie Mischungen von beliebigen zwei oder mehreren (i), (ii), (iii) oder (iv) Organophosphinarten; und

(III) mindestens ein reduzierendes Metall, ausgewählt aus der Gruppe bestehend aus Zink, Magnesium und Mangan;

wobei die Menge an zweizähnigem Phosphor-enthaltendem Liganden in den Bereich von 0,5 bis zu 20 Mol pro Gramm-atom Nickel fällt und worin die Menge an reduzierendem Metall in den Bereich von 1 bis zu 1 000 Mole pro Mol Nickel fällt.

## Revendications

1. Procédé de couplage de monohalogénures d'aryle et d'hétéroaryle en $C_4$-$C_{20}$ de façon à produire des composés biaryliques ou hétérobiaryliques de structure :

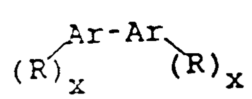

dans laquelle Ar représente une fraction aromatique en $C_4$-$C_{20}$, chaque R est choisi indépendamment parmi un groupement alkyle, un groupement aryle, -F, -NR'$_2$, -CN, -CHO, -OR', -OCO-R', -COO-R',

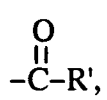

-$SO_2$-R', -$SO_3$-R' ou -NR'COR' :
où R' représente un radical hydrocarbyle ou hétéroaryle pouvant avoir jusqu'à 20 atomes de carbone, et x est un nombre entier compris dans l'intervalle de 0 à 8, selon la dimension du noyau aromatique

Ar, et à condition qu'il n'y ait pas plus d'un substituant en position ortho par rapport à la liaison Ar-Ar; ledit procédé comprenant la mise en contact dudit monohalogénure de structure :

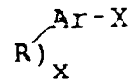

dans laquelle Ar, R et x sont tels que définis ci-dessus et X est un atome d'halogène; avec une combinaison catalytique comprenant :

(I) un composé du nickel anhydre dans lequel le rapport d'atome gramme de nickel au nombre de moles de monohalogénure est dans l'intervalle de 0,0001 à 0,5;

(II) au moins un ligand contenant du phosphore bidenté choisi dans le groupe constitué de :

(i) 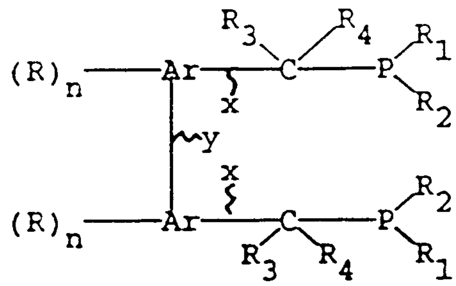

dans laquelle chaque Ar est choisi indépendamment parmi les composés à noyau aromatique en $C_6$-$C_{14}$ ;
les liaisons x et les liaisons y sont liées aux carbones adjacents aux structures aromatiques ;
chaque R, quand il est présent en tant que substituant, est choisi indépendamment entre un groupement alkyle, un groupement aryle, -F, $-NR'_2$, -CN, -CHO, -OR', -OCO-R', -COO-R',

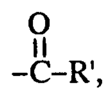

$-SO_2$-R', $-SO_3$-R' ou -NR'COR' :
où R' représente un radical hydrocarbyle ou hétéroaryle ayant jusqu'à 20 atomes de carbone ;
n est un nombre entier compris dans l'intervalle de 0 à 4, quand Ar représente un groupement phényle ; 0 à 6 quand Ar représente un groupement naphtyle et 0 à 8 quand Ar représente un groupement phénanthryle ou anthracényle ;
chaque $R_1$ et $R_2$ est choisi indépendamment parmi des radicaux alkyle, aryle, aralkyle, alkaryle ou cycloaliphatique substitués ou non substitués par des groupements amino, amido, acides sulfoniques, oxycarbonyle, hydroxyle ou alcoxy,
chaque $R_3$ et $R_4$ est choisi indépendamment entre un atome d'hydrogène et les substituants $R_1$ ;
chacun des groupements ou fractions alkyle ci-dessus est une chaîne linéaire ou ramifiée en $C_1$-$C_{20}$ ;
chaque groupement aryle contient 4 à 20 atomes de carbone de noyau ; et
chaque groupement cycloaliphatique contient 4 à 8 atomes de carbone de noyau ;

(ii) 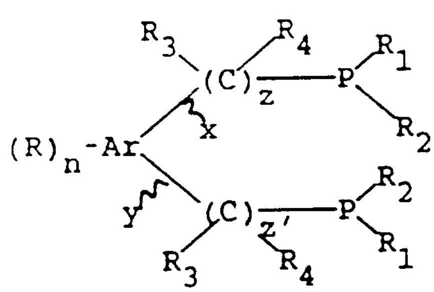

dans laquelle Ar, x, y, R, n, $R_1$, $R_2$, $R_3$ et $R_4$ sont chacun définis comme ci-dessus et z et z' peut varier chacun indépendamment entre 0 et 4, à condition que z + z' soit au moins égal à 2 ;

(iii)

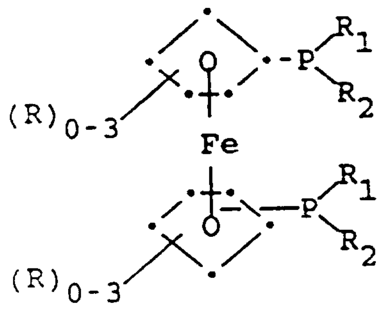

dans laquelle R, $R_1$ et $R_2$ sont tels que définis ci-dessus ; et

(iv)

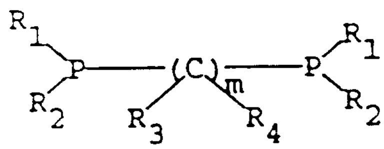

dans laquelle $R_1$, $R_2$, $R_3$ et $R_4$ sont tels que définis ci-dessus et m est un nombre entier qui peut varier de 4 à 8 ;
ainsi que des mélanges de deux quelconques ou plus de ceux-ci ; dans lequel la quantité de ligand est comprise dans l'intervalle de 0,5 à 20 mol par atome gramme de nickel ; et
(III) au moins un métal réducteur choisi dans le groupe constitué du zinc, du magnésium et du manganèse ; dans lequel le rapport molaire du métal réducteur au monohalogénure réactif est situé dans l'intervalle de 0,01 à 20 : 1 ; dans lequel ladite mise en contact est mise en oeuvre en présence d'un solvant polaire aprotique à une température dans l'intervalle de 0 à 250 ° C pendant une durée suffisante pour former lesdits composés biaryliques ou hétérobiaryliques.

**2.** Procédé selon la revendication 1, dans lequel ledit ligand bidenté a la formule de structure :

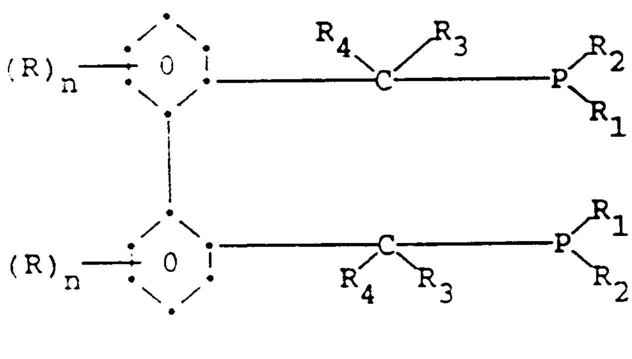

dans laquelle R, $R_1$, $R_2$, $R_3$, $R_4$ et n sont tels que définis ci-dessus.

**3.** Procédé selon la revendication 2, dans lequel ledit ligand bidenté est choisi dans le groupement constitué de :
2,2'-bis(diphénylphosphinométhyl)-1,1'-biphényle ;
2,2'-bis(dibenzylphosphinométhyl)-1,1'-biphényle ;
2,2'-bis(phénylbenzylphosphinométhyl)-1,1'-biphényle ; et
2,2'-bis(diisobutylphosphinométhyl)-1,1'-biphényle.

4. Procédé selon la revendication 1, dans lequel ledit ligand bidenté a la formule de structure :

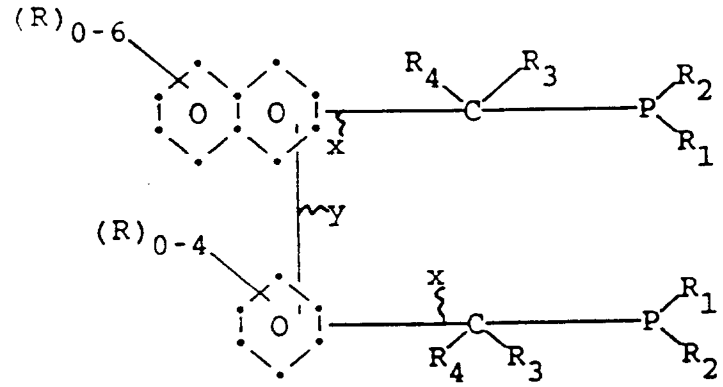

dans laquelle R, $R_1$, $R_2$, $R_3$, $R_4$, x et y sont tels que définis ci-dessus.

5. Procédé selon la revendication 4, dans lequel ledit ligand bidenté est le 2-(diphénylphosphinométhyl)-1-[2-(diphénylphosphinométhyl)phényl]naphtalène.

6. Procédé selon la revendication 1, dans lequel ledit ligand bidenté a la formule de structure :

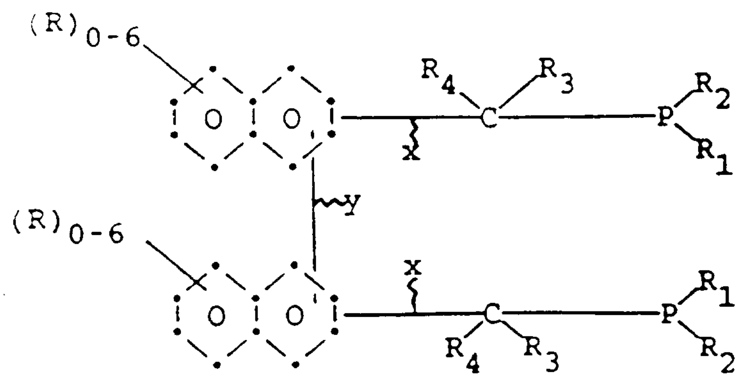

dans laquelle R, $R_1$, $R_2$, $R_3$, $R_4$, x et y sont tels que définis ci-dessus.

7. Procédé selon la revendication 6, dans lequel ledit ligand bidenté est le 2,2'-bis-(diphénylphosphinométhyl)-1,1'-binaphtyle.

8. Procédé selon la revendication 1, dans lequel ledit ligand bidenté a la structure de structure :

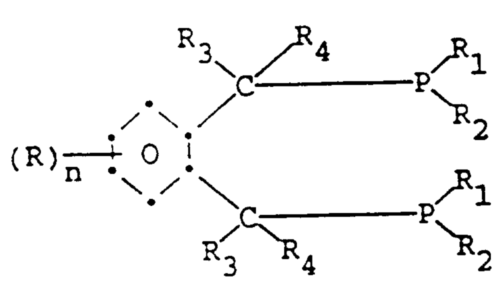

dans laquelle R, $R_1$, $R_2$, $R_3$, $R_4$ et n sont tels que définis ci-dessus.

9. Procédé selon la revendication 8 dans lequel ledit ligand bidenté est le α,α'-bis(diphénylphosphino)-ortho-xylène.

10. Procédé selon la revendication 1, dans lequel ledit ligand bidenté a la formule de structure :

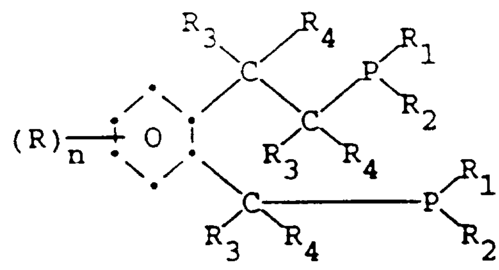

dans laquelle R, $R_1$, $R_2$, $R_3$, $R_4$ et n sont tels que définis ci-dessus.

11. Procédé selon la revendication 10, dans lequel ledit ligand bidenté est le α,β'-bis(diphénylphosphino)-2-éthyltoluène.

12. Procédé selon la revendication 1, dans lequel ledit ligand bidenté a la formule de structure :

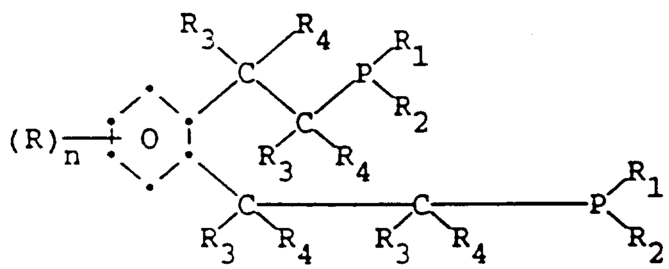

dans laquelle R, $R_1$, $R_2$, $R_3$, $R_4$ et n sont tels que définis ci-dessus.

13. Procédé selon la revendication 12, dans lequel ledit ligand bidenté est le 1,2-bis[2-(diphénylphosphino)-éthyl]benzène.

14. Procédé selon la revendication 1, dans lequel ledit ligand bidenté est le 1,1'-bis(diphénylphosphino)-ferrocène.

15. Procédé selon la revendication 1, dans lequel ledit ligand bidenté a la formule de strcture :

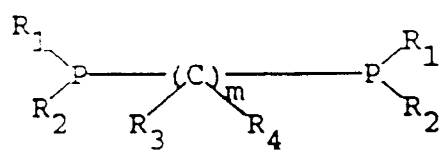

dans laquelle $R_1$, $R_2$, $R_3$ et $R_4$ sont tels que définis ci-dessus ; et dans laquelle m est un nombre entier qui peut varier de 4 à 8.

16. Procédé selon la revendication 15, dans lequel ledit ligand bidenté est choisi dans le groupe constitué de :
1,4-bis(diphénylphosphino)butane,
1,5-bis(diphénylphosphino)pentane,
1,6-bis(diphénylphosphino)hexane,

ainsi que de mélanges de deux d'entre eux ou plus.

**17.** Procédé selon l'une quelconque des revendications 1 à 16, dans lequel ladite combinaison catalytique contient de plus au moins un ligand bidenté contenant au moins un atome d'azote en tant que partie d'une structure de noyau aromatique.

**18.** Procédé selon la revendication 17, dans lequel ledit ligand bidenté contenant au moins un atome d'azote est choisi entre la 2,2'-bipyridine, une (dialkyl en $C_1$-$C_6$)aminopyridine, la phénanthroline ou l'acide 2-picolinique.

**19.** Procédé selon la revendication 1, dans lequel ledit métal réducteur est le zinc.

**20.** Procédé selon la revendication 1, dans lequel ledit composé du nickel est du chlorure de nickel.

**21.** Procédé selon la revendication 1, dans lequel on ajoute en tant qu'amorceur 0,1 mole par atome gramme de nickel d'un sel inorganique.

**22.** Procédé selon la revendication 21, dans lequel le sel inorganique est choisi dans le groupe constitué des suivants :
un iodure de métal alcalin, un bromure de métal alcalin, un chlorure de métal alcalin, ainsi que des mélanges de deux quelconques d'entre eux ou plus.

**23.** Procédé selon la revendication 1, dans lequel le solvant aprotique est le N,N-diméthylacétamide.

**24.** Procédé selon la revendication 1, dans lequel le solvant aprotique est le N,N-diméthylformamide.

**25.** Procédé selon la revendication 1, dans lequel le solvant aprotique est la 1-méthyl-2-pyrrolidinone.

**26.** Procédé selon la revendication 1, dans lequel le monohalogénure a la formule :

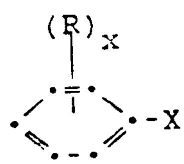

(I)

dans laquelle un ou plusieurs des atomes de carbone du noyau benzène de la formule (I) peut ou peuvent éventuellement être remplacé par N ;
dans laquelle X est un atome d'halogène choisi dans le groupe constitué de Cl, Br et I ;
R est un radical monovalent choisi entre un groupement alkyle, un groupement aryle, -F, $-NR'_2$, -CN, -CHO, -OR', -OCO-R', -COO-R',

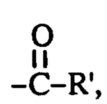

$-SO_2$-R', - $SO_3$-R' ou -NR'COR' :
dans lesquelles R' est un radical hydrocarbyle ou hétéroaryle ayant jusqu'à 20 atomes de carbone, et x représente un nombre entier valant de 0 à 4, à condition qu'il n'y ait pas plus d'un R en position ortho par rapport à l'atome de carbone contenant X.

**27.** Procédé selon la revendication 26, dans laquelle X est Cl.

**28.** Procédé selon la revendication 27, dans lequel ledit monohalogénure est choisi dans le groupe constitué des suivants :
2-chlorotoluène,
2-chloropyridine
4-fluoro-2-chlorotoluène,
éther méthylique de 2-chlorobenzyle,
éther éthylique de 2-chlorobenzyle,
4-(N-éthyl-N-acétyl)amino-2-chlorotoluène,
6-(N-éthyl-N-acétyl)amino-2-chlorotoluène,
4-chlorotoluène,
4-chloroanisole,
éther (2-méthoxy)éthylique de 2-chlorobenzyle,
ainsi que des mélanges de deux quelconques ou plus de ceux-ci.

**29.** Procédé selon la revendication 26, dans lequel R représente un groupement méthyle et x vaut 1.

**30.** Procédé selon la revendication 1, dans lequel la température est comprise entre 25 ° C et 120 ° C.

**31.** Procédé selon la revendication 30, dans lequel le rapport des atomes gramme de nickel aux moles de monohalogénure se situe dans l'intervalle de 0,01 à 0,2 : 1; la quantité de ligand bidenté contenant du phosphore se situe dans l'intervalle de 1 à 10 moles par atome gramme de nickel ; et le rapport molaire du métal réducteur au monohalogénure réactif se situe dans l'intervalle de 0,2 à 10 : 1.

**32.** Procédé selon la revendication 30, dans lequel le rapport des atomes gramme de nickel aux moles de monohalogénure se situe dans l'intervalle de 0,03 à 0,1 : 1 ; la quantité de ligand contenant du phosphore bidenté se situe dans l'intervalle de 1 à 5 moles par atome gramme de nickel ; et le rapport molaire du métal réducteur au monohalogénure réactif se situe dans l'intervalle de 0,4 à 5 : 1.

**33.** Combinaison catalytique active comprenant :
(I) un composé du nickel anhydre,
(II) au moins un ligand bidenté contenant du phosphore choisi dans le groupe constitué de :

(i) 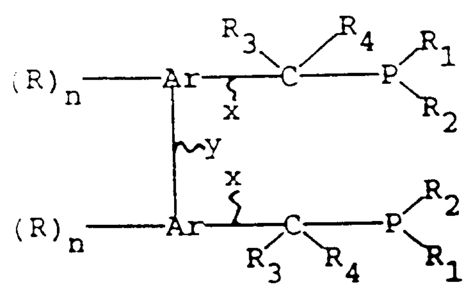

dans laquelle chaque Ar est choisi indépendamment parmi des composés à noyaux aromatiques en $C_6$-$C_{14}$ ;
les liaisons x et les liaisons y sont liées aux carbones adjacents aux structures à noyau ;
chaque R, quand il est présent en tant que substituant, est choisi indépendamment entre un groupement alkyle, un groupement aryle, -F, -NR'$_2$, -CN, -CHO, -OR', -OCO-R', -COO-R',

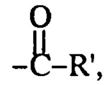

-SO$_2$-R', -SO$_3$-R' ou -NR'COR' :
où R' représente un radical hydrocarbyle ou hétéroaryle ayant jusqu'à 20 atomes de carbone ;
n représente un nombre entier compris dans l'intervalle de 0 à 4 quand Ar représente un groupement phényle ; 0 à 6 quand Ar représente un groupement naphtyle et 0 à 8 quand Ar

représente un groupement phénanthryle ou anthracényle ;
chaque $R_1$ et $R_2$ est choisi indépendamment parmi des radicaux alkyle, aryle, aralkyle, alkaryle ou cycloaliphatique substitués ou non substitués par des groupements amino, amido, acides sulfoniques, oxycarbonyle, hydroxyle ou alcoxy, chaque $R_3$ et $R_4$ est choisi indépendamment entre un atome d'hydrogène et les substituants $R_1$ ;
chacun des groupements ou fractions alkyle ci-dessus est une chaîne linéaire ou ramifiée en $C_1$-$C_{20}$ ;
chaque groupement aryle contient 4 à 20 atomes de carbone de noyau ; et
chaque groupement cycloaliphatique contient 4 à 8 atomes de carbone de noyau;

(ii)

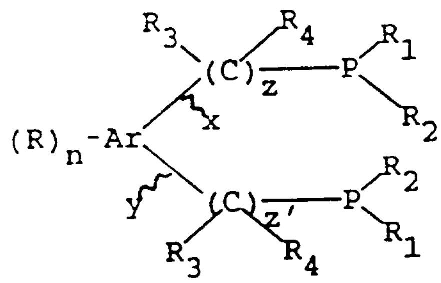

dans laquelle Ar, x, y, R, n, $R_1$, $R_2$, $R_3$ et $R_4$ sont chacun définis comme ci-dessus, et z et z' peuvent chacun indépendamment varier entre 0 et 4 à condition que z + z' soit au moins égal à 2 ;

(iii)

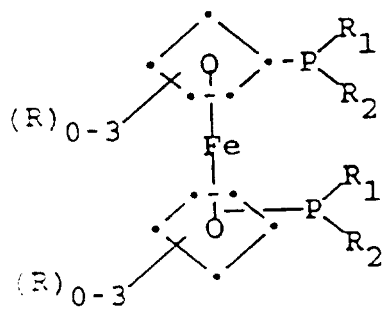

dans laquelle R, $R_1$ et $R_2$ sont tels que définis ci-dessus et

(iv)

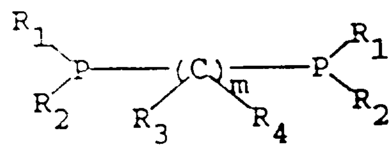

dans laquelle $R_1$, $R_2$, $R_3$ et $R_4$ sont tels que définis ci-dessus et dans laquelle m représente un nombre entier qui varie de 5 à 8 ;
ainsi que des mélanges de deux quelconques ou plus desdites organophosphines de type (i), (ii), (iii) ou (iv) ; et
(III) au moins un métal réducteur choisi dans le groupe constitué du zinc, du magnésium et du manganèse ;
dans lequel la quantité de ligants contenant du phosphore bidenté se situe dans l'intervalle de 0,5 à 20 moles par atome gramme de nickel et dans lequel la quantité de métal réducteur se situe dans l'intervalle de 1 à 1 000 moles par mole de nickel.